(19) Europäisches Patentamt
European Patent Office
Office européen des brevets

(11) Publication number: 0 052 946 A1

# (12) EUROPEAN PAENT APPLICATION

(21) Application number: 81305033.3

(51) Int. Cl.$^3$: C 08 L 67/04

(22) Date of filing: 26.10.81

(30) Priority: 20.11.80 GB 8037256
05.02.81 GB 8103497
05.02.81 GB 8103496

(43) Date of publication of application:
02.06.82 Bulletin 82/22

(84) Designated Contracting States:
BE CH DE FR GB IT LI NL SE

(71) Applicant: IMPERIAL CHEMICAL INDUSTRIES PLC
Imperial Chemical House Millbank
London SW1P 3JF(GB)

(72) Inventor: Henman, Terence John
2 Meadow Way
Melbourn Cambridgeshire(GB)

(72) Inventor: Holmes, Paul Arthur
39 Skottowe Crescent
Great Ayton Middlesbrough Cleveland(GB)

(74) Representative: Downer, John Michael et al,
Imperial Chemical Industries PLC Legal Department:
Patents Thames House North Millbank
London SW1P 4QG(GB)

## (54) Stabilised polyester compositions.

(57) Microbiologically produced polymers based on β-hydroxybutyric acid of improved melt stability containing a polyethylenically unsaturated compound containing at least two reactive groups of the formula $-CH=CR_1-$ in which $R_1$ is hydrogen, a lower alkyl or a phenyl radical and in which compound $R_1$ may differ from one $-CH=CR_1-$ group to another with the proviso that where the compound contains only two groups of the formula $-CH=CR_1-$, they are not both contained in:

R–CH=CR$_1$–C–O–
              ||
              O

groups where R is hydrogen, a lower alkyl, or a phenyl radical. The polyethylenically unsaturated compounds are most effective when used in the presence of free radical generating agents, although thermal and shear induced activation may be employed. Preferred ethylenically unsaturated compounds contain at least two allyl compounds, at least two maleimido groups or at least three groups of formula:

R–CH=C–C–O–
          |   ||
          R$_1$ O

EP 0 052 946 A1

STABILISED POLYESTER COMPOSITIONS

This invention relates to biologically produced poly(β-hydroxybutyric acid) compositions having improved thermal stability and to methods for the production thereof.

Poly(β-hydroxybutyric acid) is a thermoplastic polyester consisting of repeat units of the formula:

$$-CH(CH_3).CH_2.CO.O-$$

which is accumulated by many micro-organisms, particularly bacteria, for example of the genera Alcaligenes, Athiorhodium, Azotobacter, Bacillus, Nocardia, Pseudomonas, Rhizobium, and Spirillium, as an energy reserve material.

The polymer is conveniently prepared by cultivating the micro-organism in an aqueous medium on a suitable substrate, such as a carbohydrate or methanol, as an energy and carbon source. The substrate must, of course, be one that is assimilable by the micro-organism. In order to promote accumulation of the polymer, at least part of the cultivation is preferably conducted under conditions wherein there is a limitation of a nutrient that is essential for growth of the micro-organism but which is not required for polymer accumulation. Examples of the suitable processes are described in European Patent Specification 15669 and in European Patent Application 81303373.

Polymers containing both β-hydroxybutyric acid units and other hydroxycarboxylic acid units, such as β-hydroxyvaleric acid units, can also be produced microbiologically. Thus a microbiologically produced heteropolymer containing β-hydroxybutyric acid and β-hydroxyvaleric acid residues is described by Wallen et al in "Environmental Science and Technology" 8

(1974) 576-9. Also, as described in United Kingdom Patent Application 8120991 various copolymers can be produced by cultivating the micro-organism on certain substrates, such as propionic acid which gives rise to β-hydroxyvaleric acid units in the copolymer.

While cells containing the polymer can be used as such as a moulding material, for example as described in USP 3 107 172, it is generally desirable to separate the polymer from the remainder of the cell material.

Methods that have been proposed to effect this separation include breakage of the cells by methods such as treatment with acetone, followed by extraction of the polymer from the broken cells by treatment with a solvent in which the polymer is soluble. Examples of such processes are described in USP's 3 036 959 and 3 044 942 in which the solvents employed are pyridine or mixture of methylene chloride and ethanol. Other extraction solvents for the polymer in the form in which it is produced in the cells include cyclic carbonates such as 1,2-propylene carbonate (see USP 4 101 533); chloroform (see USP 3 275 610); and 1,2-dichloroethane (as disclosed in European Patent Specification 15123).

USP 3 275 610 discloses other methods of cell breakage viz. ultrasonic vibration, grinding, French pressing, freezing/thawing cycles and lysozyme treatment, while, as described in the aforementioned European Patent Specification 15123, spray or flash drying of the suspension of cells as produced by culturing the micro-organism can also cause sufficient cell breakage to enable the polymer to be extracted from the cells.

The polymer extracted from the micro-organism tends to exhibit discoloration or browning when subjected to shaping operations, particularly those involving heat. While this discoloration may be reduced by purification of the polymer, for example by removal of cell debris, e.g.

by filtration of a solution of the polymer, and by removal of lipids, before or after separation of the polymer from the cells, e.g. by extraction with a solvent in which the lipids are soluble but in which the polymer is insoluble, the polymer tends to degrade on exposure to elevated temperatures, e.g. during melt shaping processes such as compression moulding, injection moulding, and extrusion.

Copolymers can also be made containing units of other hydroxycarboxylic acids and/or units derived from diols, e.g. ethylene glycol, and/or dicarboxylic acids, e.g. isophthalic acid, by ester interchange occurring when the microbiolically produced polymer or copolymer is melted with such a hydroxycarboxylic acid, lactone thereof, e.g. pivalolactone, diol, dicarboxylic acid and/or polyester produced therefrom.

Such copolymers prepared by ester interchange are similarly liable to thermal degradation.

We have now found that this thermal degradation of such β-hydroxybutyric acid unit containing polymers, hereinafter referred to as HB polymers, can be substantially reduced.

In the following description therefore by the term "HB polymer" we mean not only the homopolymer of β-hydroxybutyric acid but also copolymers as described above containing at least 50 mole %, and preferably at least 80 mole % of β-hydroxybutyric acid residues in the polymer chain.

According to the invention there is provided an HB polymer composition characterised in that it contains an HB polymer and a sufficient quantity of a polyethylenically unsaturated compound containing at least two reactive groups of the formula $-CH=CR_1-$ in which $R_1$ is hydrogen, a lower alkyl or a phenyl radical and in which compound $R_1$ may differ from one $-CH=CR_1-$ group to another with the proviso that where the compound

contains only two groups of the formula $-CH=CR_1-$, they are not both contained in:

$$R-CH=CR_1-\underset{\underset{O}{\|}}{C}-O-$$

groups where R is hydrogen, a lower alkyl, or a phenyl radical to improve the melt stability of the HB polymer. By lower alkyl radical is meant radicals containing 1 to 4 carbon atoms.

Surprisingly, polyethylenically unsaturated compounds containing reactive groups of the above formula are more effective stabilisers than, for example, compounds containing two groups of the formula:

$$R-CH=CR_1-\underset{\underset{O}{\|}}{C}-O- \quad ,$$

for example two acrylate or methacrylate groups.

Improved results are generally obtained when the composition also contains a free-radical-generating agent although the polyethylenically unsaturated compounds may be effectively activated by elevated temperatures or by shear such as experienced in melt mixing in an extruder.

The composition may consist simply of a physical blend of the components of the composition so that any chemical interaction between the HB polymer and the polyethylenically unsaturated compounds only takes place when the composition is melted during or prior to fabrication. In such a case the polyethylenically unsaturated compound should have only limited volatility at the temperature of the melt so that it is not volatilised from the melt. Alternatively, the composition may already have been melt compounded so that the composition consists of an HB polymer which has been chemically modified by the polyethylenically unsaturated compound. In this case the polyethylenically unsaturated

compound used may have greater volatility because the melt can be compounded in a closed system to give the chemically modified polymer. Whatever method is chosen it is desirable to achieve the maximum degree of dispersion of the additive in the polymer melt to ensure the most effective use of the additive.

Accordingly there is also provided an HB polymer composition characterised in that it has been prepared by reacting an HB polymer with at least one polyethylenically unsaturated compound as hereinbefore defined, in a melt of the HB polymer optionally in the presence of a free-radical generating agent.

The improvement in stability brought about by the addition of the specified additives may be measured by the effect the additives have on the melt viscosity of the HB polymer. This may conveniently be assessed by measuring the change in melt flow time through a capillary die.

By a "melt flow time" is meant the time taken for a 1 gram sample of the HB polymer to flow through a die of length 8 mm and diameter 2.095 mm under a load of 2.16 kg at a temperature of 190°C ± 1°C after a heating up period of 5 minutes. This method of assessment is substantially as described in British Standard 2782:7:720A (1979).

The melt flow time is a measure of the thermal stability of the HB polymer. Microbiologically produced HB polymers which have subsequently been purified by the known methods have a melt flow time of 4 to 6 minutes. The polymers resulting from reaction with the specified additives have melt flow times which are significantly above these values.

Accordingly in a further embodiment of the invention there is provided a modified biologically produced HB polymer composition having a melt flow time which is at least double that of the HB polymer itself.

Polyethylenically unsaturated compounds are known in the art and are widely used as cross-linking agents in a variety of polymers. They are most usually used by incorporation during the process of polymerising the said polymers. However the HB polymer compositions of the present invention are produced by the addition of the polyethylenically unsaturated compounds to a preformed HB polymer.

One class of polyethylenically unsaturated compounds which may be used have at least two addition polymerisable unsaturated groups of the formula:

$$RCH{=}\underset{\displaystyle R_1}{\underset{|}{C}}{-}CHR_2{-}$$

where R and $R_1$ may be the same or different and are selected from hydrogen, lower alkyl or phenyl radicals and $R_2$ may be a hydrogen or lower alkyl radical. It is preferred that at least three addition polymerisable unsaturated groups be present in the compound. These groups may be such that they have equal reactivity, that is, they will react at the same rate as each other in free radical reactions, or they may have substantially different reactivities.

Typical materials of this type are compounds containing two or more allyl, methallyl and crotonyl radicals such as diallyl fumarate, triallyl phosphate, triallyl cyanurate and isocyanurate, tetra-(allylhydroxy)ethane, diallyl succinate, diallyl terephthalate, diallyl carbonate, ethylene glycol bis(allyl carbonate), di-, tri- and tetra-ethylene glycol bis(allyl carbonates), glyceryl trisallyl carbonate, ethylene glycol bis(2-chloroallyl carbonate) and corresponding methallyl derivatives of these carbonates.

Other suitable materials within the class of compounds containing at least two addition polymerisable unsaturated groups of the formula specified are compounds containing a single group of formula:

$$R\text{-}CH{=}\underset{\substack{|\\R_1}}{C}\text{-}CHR_2\text{-}$$

where R and $R_1$ may be the same or different and are selected from hydrogen, lower alkyl or phenyl radicals and $R_2$ may be hydrogen or lower alkyl, together with at least one different ethylenically unsaturated group such as a group of formula:

$$R\text{-}CH{=}CR_1\text{-}\underset{\substack{\|\\O}}{C}\text{-}O\text{-}$$

(where R and $R_1$ are as previously specified) for example, allyl acrylate, allyl methacrylate, methallyl acrylate and methallyl methacrylate.

Although compounds containing only two groups of formula $-CH{=}CR_1-$, both of which are:

$$R\text{-}CH{=}CR_1\text{-}\underset{\substack{\|\\O}}{C}\text{-}O\text{-}$$

groups are not included within the invention because they are not sufficiently effective in improving the melt stability of PHB, additives containing at least three groups of formula:

$$R\text{-}CH{=}CR_1\text{-}\underset{\substack{\|\\O}}{C}\text{-}O\text{-}$$

are included within the invention. Specific compounds of this type which are suitable for use in the invention are pentaaerythrityl triacrylate, pentaaerythrityl monohydroxypentaacrylate, and glyceryl and trimethylolpropane triacrylates.

The HB polymer composition should generally be processed at a temperature above the melting point of both the polyethylenically unsaturated compound and the HB polymer.

By the melting point of the HB polymer we mean the temperature of the peak in the melting endotherm as determined by differential scanning calorimetry. Where the HB polymer exhibits two or more such peaks, as in the case of certain HB copolymers, the melting point is deemed to be the temperature of that peak occurring at the higher, or highest, temperature.

Particularly useful are polyethylenically unsaturated compounds which have a melting point close to the processing temperature of the HB polymer. Thus compounds which have a melting point which is within the range from about 20°C below the melting point of the HB polymer, which is about 180°C for β-hydroxybutyric acid homopolymer, to about 20°C above the melting point of the HB polymer are particularly suitable for use in the invention. Preferably the polyethylenically unsaturated compound should have a melting point within the range from about 0-10°C below the melting point of the HB polymer. A material which meets these requirements is the allyl substituted diamide, diallyl tartardiamide.

Other substituted amides of formula:

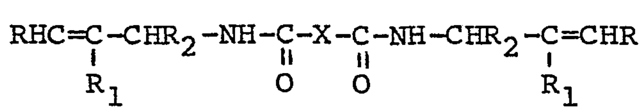

where R, $R_1$ and $R_2$ are as specified previously and -HN-OC-X-CO-NH- is a residue of a diamide, may be used, particularly those having a melting point just below that of the HB polymer.

Another class of polyethylenically unsaturated compounds containing at least two reactive groups of the formula $-CH=CR_1-$ which have been found to be effective are the maleimides, particularly the dimaleimides of formula:

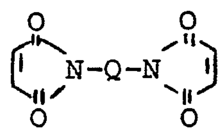

where Q is a divalent aliphatic, aromatic or cycloaliphatic radical. Typical examples of suitable maleimides are N,N'-ethylene dimaleimide, N,N'-p-phenylene dimaleimide, N,N'-m-phenylene dimaleimide and 4,4'-bismaleimide diphenyl methane.

The polyethylenically unsaturated compounds are preferably present in the composition of the invention at a concentration of 0.1 to 10% by weight and desirably within the range 0.5 to 5.0% by weight.

These compounds have been found to be most effective when used in the presence of a free-radical-generating agent which will generate free radicals at temperatures at which the HB polymer melts.

Suitable free radical generators will always have a half life of >0.01 hour at 130°C, but preferred materials have half lives at 130°C, of at least 1.0 hours.

Suitable classes of initiator include a wide range of peroxides such as hydroperoxides, dialkyl peroxides, peresters, perketals, ketone peroxides, acyl peroxides, percarbonates and mixtures of these.

As examples of hydroperoxides there may be mentioned: tert-butyl hydroperoxide; tert-amyl hydroperoxide; 1,1,3,3-tetramethylbutyl hydroperoxide; cumene hydroperoxide; di-isopropyl benzene hydroperoxide; para-menthane hydroperoxide; pinane hydroperoxide; 2,5-dimethyl-2,5-dihydroperoxy hexane; and 2,5-dimethyl-2,5-dihydroperoxy hex-3-yne.

As examples of dialkyl peroxides there may be mentioned: di-tert-butyl peroxide; di-tert-amyl peroxide; tert-amyl tert-butyl peroxide; butyl-2-hydroxyethyl peroxide; di-tert-butyl decamethylene bisperoxide; tert-butylcumyl peroxide; dicumyl peroxide; α,α'-bis(tert-butylperoxy)di-isopropyl benzene; 2,5-dimethyl-2,5-di-tert-butylperoxy hexane; 2,5-dimethyl-2,5-di-tert-butylperoxy hex-3-ene; and 2,5-dimethyl-2,5-di-tert-butylperoxy hex-3-yne.

As examples of peresters there may be mentioned: tert-butyl peroctoate; tert-butyl perpivalate; tert-butyl perisononanoate; tert-butyl perneodecanoate; tert-butylperoxy crotonate; di-tert-butyldiperoxy succinate; 1,1,3,3-tetramethylbutylperoxy-2-ethyl hexanoate; tert-butyl perbenzoate; tert-butyl monoperphthalate; di-tert-butyldiperoxyphthalate; phthalide peroxide; 2,5-dimethyl hexane-2,5-diperbenzoate; and 2,5-dimethyl hexane-2,5-peroctanoate.

As examples of perketals there may be mentioned: 2,2-bis(tert-butylperoxy)butane; 2,4-pentane dione peroxide; 1,1-bis(tert-butylperoxy)cyclohexane; and 3,3,5-trimethyl-1,1-bis(tert-butylperoxy)cyclohexane.

As examples of ketone peroxides there may be mentioned: methyl ethyl ketone peroxide; methyl isobutyl ketone peroxide; methyl amyl ketone peroxide; acetyl acetone peroxide; and bis(1-hydroxycyclohexyl)peroxide.

As examples of acyl peroxides there may be mentioned: lauroyl peroxide; benzoyl peroxide; di(4-chlorobenzoyl)peroxide; and bis(2,4-dichlorobenzoyl peroxide.

As an example of a percarbonate there may be mentioned: tert-butyl peroxyisopropyl carbonate.

These may be present at lower concentrations than the polyfunctional additives, preferably from 0.01 to 2.0% by weight of the total composition.

The performance of the free radical generators may be advantageously modified by the inclusion of free radical inhibitors such as para-tert-butyl catechol.

The optimum amounts of ingredients are assessed by assessing the thermal stability of the composition as measured by the melt flow index of the composition relative to the unstabilised composition at a given temperature at which the HB polymer will be molten since the melt flow index of the composition can change within the time scale of the measurement, a convenient parameter for assessing stabilisers is the flow time of a standard weight sample. The quantities of stabilising ingredients present should be sufficient to increase the flow time compared to that of the unstabilised polymer by a factor of 2 or more and preferably by a factor of 4 or more when assessed according to the method hereinbefore defined.

The invention is further illustrated by reference to the following examples.

Throughout the examples the proportions of additives are expressed as % by weight of the total composition.

EXAMPLE 1

Samples containing 1 g of dry a powdered HB polymer and the additives shown in Table 1 were blended in 2 ml of methylene chloride and dried in an oven at 60°C for 30 minutes.

The blend was then rapidly charged into a standard melt flow grader (made by Davenports, Welwyn Garden City, UK) fitted with a standard die (length 8 mm, diameter

2.095 mm) and pre-heated to 190°C ± 0.5°C. After 5 minutes warming-up time, the contents of the grader were extruded using a 2 kg weight on the piston (weight 0.16 kg) and the total flow time noted.

A control sample of the HB polymer (without additives) was similarly treated and the % increase in flow time for the blended samples was calculated with respect to the flow time of the control. The results are given in Table 1.

The HB polymer was a β-hydroxybutyric acid homopolymer produced by aerobic cultivation of Alcaligenes eutrophus mutant S301/C5 (NCIB 11599) in an aqueous medium using glucose as a substrate. The polymer was isolated by extraction from the aqueous cell suspension with 1,2-dichloroethane at room temperature followed by separation of the solvent layer, containing the dissolved polymer, from the aqueous layer by decanting. The solution was filtered and then the polymer was precipitated by adding the solution to a methanol/water mixture. The precipitated polymer was separated by filtration and dried.

TABLE 1

| Polyfunctional additive (% by weight of polymer) | Peroxide (% by weight of polymer) | Flow time (mins.) | % Increase |
|---|---|---|---|
| NIL | NIL | 5 | - |
| 1.0 TAC* | 0.1 DCP** | 55 | 1000 |
| 1.5 TAC | 0.5 DCP | 85 | 1600 |

* TAC = triallyl cyanurate.
** DCP = dicumyl peroxide.

EXAMPLE 2

The procedure of Example 1 was repeated using a mixture of diethylene glycol bis(allyl carbonate) (1% by weight) and dicumyl peroxide (0.1% by weight). A melt flow time of 15 minutes was obtained for a 1 gram sample of the HB polymer composition.

COMPARATIVE EXAMPLE A

The procedure of Example 1 was repeated using a monoethylenically unsaturated compound, methyl methacrylate, for comparative purposes. Using 1% by weight of methyl methacrylate and 0.1% by weight of dicumyl peroxide a melt flow time of 7 minutes was obtained for 1 gram of the HB polymer composition.

COMPARATIVE EXAMPLE B

The procedure of Example 1 was repeated using a variety of diacrylates and dimethacrylates as listed in Table 2.

TABLE 2

| Polyfunctional additive (% by weight of polymer) | Peroxide (% by weight of polymer) | Flow time (seconds) |
|---|---|---|
| Control | - | 300 |
| 1.0 triethylene glycol dimethacrylate | 0.1 DCP | 275 |
| 1.0 tetraethylene glycol dimethacrylate | 0.1 DCP | 245 |
| 1.0 polyethylene glycol 200 dimethacrylate | 0.1 DCP | 262 |
| 1.0 diethylene glycol dimethacrylate | 0.1 DCP | 262 |
| 1.0 1,6-hexane diol diacrylate | 0.1 DCP | 582 |
| 1.0 1,3-butane diol dimethacrylate | 0.1 DCP | 210 |

EXAMPLE 3

The procedure of Example 1 was repeated except in that the triallyl cyanurate was replaced by a variety of allyl substituted compounds as listed in Table 3.

TABLE 3

| Polyfunctional additive (% by weight of polymer) | Peroxide (% by weight of polymer) | Flow time (minutes) |
|---|---|---|
| Control | - | 5 |
| 1.0 diallyl succinate | 0.1 DCP | 23 |
| 1.0 triallyl phosphate | 0.1 DCP | 25 |
| 1.0 diallyl carbonate | 0.1 DCP | 37 |
| 1.0 diallyl terephthalate | 0.1 DCP | 39 |
| 1.0 triallyl isocyanurate | 0.1 DCP | 41 |

EXAMPLE 4

The procedure of Example 1 was repeated except in that the triallyl cyanurate was replaced by a variety of compounds containing more than two acrylate groups. The results are given in Table 4.

TABLE 4

| Polyfunctional additive (% by weight of polymer) | Peroxide (% by weight of polymer) | Flow time (seconds) | % Increase |
|---|---|---|---|
| NIL | NIL | 300 | - |
| A | 0.1 DCP | 900 | 200 |
| B | 0.1 DCP | 937 | 212 |
| C | 0.1 DCP | 990 | 230 |

Notes to Table 4

A = pentaerythrityl triacrylate.

B = pentaerythritylmonohydroxypentaacrylate.

C = pentaerythrityl tetraacrylate.

DCP = dicumyl peroxide.

EXAMPLE 5

The procedure of Example 1 was repeated to evaluate the effect of various concentrations of triallylcyanurate and dicumyl peroxide. The results are tabulated in Table 5.

TABLE 5

| Concentration of TAC (%) | Concentration of DCP (%) | Flow time (minutes) |
|---|---|---|
| 0.1 | 0.1 | 18 |
| 0.25 | 0.1 | 26 |
| 0.25 | 0.5 | 29 |
| 0.5 | 0.1 | 42.5 |
| 0.5 | 0.25 | 43 |
| 0.5 | 0.5 | 41 |
| 1.0 | 0.25 | 65 |
| 1.0 | 0.5 | 57.5 |
| 1.0 | 1.0 | 60.5 |
| 1.5 | 0.1 | 69.0 |
| 1.5 | 0.25 | 67.5 |
| 1.5 | 0.5 | 78 |
| 1.5 | 1.0 | 82.5 |
| 2.0 | 0.25 | 94 |

EXAMPLE 6

The procedure of Example 1 was used to evaluate a number of bismaleimides in the presence of 0.1% dicumyl peroxide and other peroxides. The results are given in Table 6.

TABLE 6

| Additive Concentration (%) | Peroxide Concentration (%) | Flow time (minutes) |
|---|---|---|
| 1.0 ethylene bismaleimide | 0.1 DCP | 25 |
| 1.0 N,N'-m-phenylene bismaleimide | 0.1 DCP | 16 |
| 1.0 4,4'-bismaleimide diphenyl methane | 0.1 DCP | 14 |
| 1.0 N,N'-phenylene bismaleimide | 0.1 DCP | 12 |
| 1.0 ethylene bismaleimide | 0.1 Trigonox M50* | 8 |
| 1.0 ethylene bismaleimide | 0.1 Trigonox K80** | 27 |

* Trigonox M50 = methyl ethyl ketone hydroperoxide.

** Trigonox K80 = cumene hydroperoxide.

EXAMPLE 7

The procedure of Example 1 was used to evaluate the effect of the allyl substituted diamide, N',N'-diallyl tartardiamide (DATDA). The results obtained are recorded in Table 7.

TABLE 7

| Additive Concentration (%) | Peroxide Concentration (%) | Flow time (minutes) |
| --- | --- | --- |
| 1.0 DATDA | 1.0 cumene hydroperoxide | 13 |
| 1.0 DATDA | 2.0 cumene hydroperoxide | 21 |
| 2.0 DATDA | 2.0 cumene hydroperoxide | 13 |
| 1.0 DATDA | 0.5 di-tert-butyl peroxide | 18 |
| 1.0 DATDA | 0.5 methyl ethyl ketone peroxide | 18 |

EXAMPLE 8

The procedure of Example 1 was used to evaluate the monoallyl compound allyl methacrylate. The composition contained 1.0% allyl methacrylate and 0.1% dicumyl peroxide and gave a flow time of 510 seconds compared with 290 seconds for the control polymer.

EXAMPLE 9

This example shows the effect of polyethylenically unsaturated compounds on the melt stability of HB copolymers. The copolymers were prepared as for the homopolymer described in Example 1 except that when the aqueous fermentation medium became depleted in nitrogen source, the glucose substrate was replaced by propionic acid (for samples A and B) or by a mixture of propionic

acid and glucose (for sample C) and cultivation continued.

The copolymers were found to contain β-hydroxyvaleric acid units in the following concentrations:

| | |
|---|---|
| A | 4 (mole %) |
| B | 6 |
| C | 7 |

The products obtained were evaluated according to the procedure of Example 1 except in that sample C was evaluated at a melt temperature of 180°C rather than 190°C. The results obtained are shown in Table 8.

TABLE 8

| Polymer Sample | Polyethylenically unsaturated compound (%) | Peroxide (%) | Flow time (minutes) |
|---|---|---|---|
| A | NIL | NIL | 4 |
| A | 1.0 triallyl isocyanurate | 0.1 dicumyl peroxide | 13 |
| A | 1.0 N,N'-ethylene bismaleimide | 0.5 di-tert-butyl peroxide | 18 |
| B | NIL | NIL | 2 |
| B | 1.0 triallyl isocyanurate | 0.1 dicumyl peroxide | 11 |
| B | 1.0 N,N'-ethylene bismaleimide | 0.5 di-tert-butyl peroxide | 15 |
| C* | NIL | NIL | 15 |
| C* | 1.0 triallyl isocyanurate | 0.1 dicumyl peroxide | 24 |
| C* | 1.0 N,N'-ethylene bismaleimide | 0.5 di-tert-butyl peroxide | 30 |

C* This polymer sample contained 1% by weight of talc.

EXAMPLE 10

A number of compounds containing a single allyl group and no other ethylenic unsaturation were evaluated in the manner described in Example 1 in an HB homopolymer. The results recorded below in Table 9 show that no significant increase in melt flow time is obtained.

TABLE 9

| Additive Concentration (%) | Peroxide Concentration (%) | Flow time (minutes) |
|---|---|---|
| NIL | NIL | 5 |
| 1.0 allyl ethyl ether | 0.1 DCP | 5 |
| 1.0 allyl laurate | 0.1 DCP | 6 |
| 1.0 allyl oxyethanol | 0.1 DCP | 4 |

EXAMPLE 11

In order to evaluate some of the better stabilisation systems under conditions that were more relevant to commercial processing operations, a series of multiple extrusion tests were performed.

The polyethylenically unsaturated compound and free radical generator were dry blended with fibrous HB homopolymer. The mixture was mixed into a thick paste with 10% w/v chloroform and densified. After drying the resulting pellets their melt stability was determined as described in Example 1 except in that the flow time for 3 g samples was recorded rather than for 1 g samples.

The compositions were then melt extruded to form a 4 mm diameter lace in a 1" diameter extruder (Betol 2520) using a temperature profile of 165°C-180°C-200°C-200°C and

a screw speed of 60 rpm. The extruded lace was crystallised in a water bath at 60°C and pelletised using a standard lace cutter. The resulting granules were dried overnight in a vacuum oven at 60°C and their melt stability evaluated using the standard melt flow test described in Example 1.

The granules were then re-extruded and re-pelletised under the conditions described above and, after drying, the melt stability of the twice extruded polymer determined by the standard test.

This procedure was repeated until the polymer had been extruded a total of five time. The results are given in Table 10.

TABLE 10

| Additives (%) | Flow time (minutes) | | | | | |
|---|---|---|---|---|---|---|
| | Before Extrusion | After 1st Extrusion | After 2nd Extrusion | After 3rd Extrusion | After 4th Extrusion | After 5th Extrusion |
| 1% TAC + 1% TRIG. K80* | 6 | 40 | 40 | 30 | 35 | 32 |
| 1% EDMI + 0.5% TRIG. K80 | 16 | 14 | 14 | 14 | 15 | 15 |
| 1% TAP + 0.5% TRIG. K80 | 11 | 13 | 6 | 6 | 11 | 20 |
| 1% DAS + 0.1% DCP | 15 | 13 | 11 | 11 | 10 | 10 |
| 2% DATDA + 2% TRIG. K80* | 14 | 16 | 16 | 15 | 15 | 14 |
| NIL (CONTROL) | 9 | 7 | 7 | 7 | 6 | 5 |

* These samples were graded with 10 kg load rather and 2 kg load.

DAS = diallyl succinate
EDMI = ethylene di-bismaleimide
DATDA = diallyl tartardiamide
TRIG. K80 = cumene hydroperoxide
DCP = dicumyl peroxide
TAP = triallyl phosphate

EXAMPLE 12

A sample of HB polymer was dry blended with 1% by weight of triallyl cyanurate. The composition was evaluated using the melt flow grader procedure described in Example 1. A melt flow time of 20 minutes was obtained for the composition compared with a value of 7 minutes for the HB polymer not containing the additive.

CLAIMS

1. An HB polymer composition characterised in that it contains an HB polymer and a sufficient quantity of a polyethylenically unsaturated compound containing at least two reactive groups of the formula $-CH=CR_1-$ in which $R_1$ is hydrogen, a lower alkyl or a phenyl radical and in which compound $R_1$ may differ from one $-CH=CR_1-$ group to another with the proviso that where the compound contains only two groups of the formula $-CH=CR_1-$, they are not both contained in:

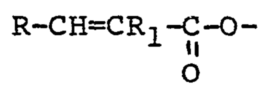

groups where R is hydrogen, a lower alkyl, or a phenyl radical to improve the melt stability of the HB polymer.

2. An HB polymer composition according to claim 1 in which the polyethylenically unsaturated compound contains at least two groups of the formula:

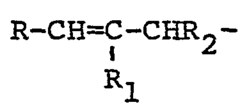

where R and $R_1$ may be the same or different and are selected from hydrogen, lower alkyl or phenyl radicals and $R_2$ may be hydrogen or lower alkyl radical.

3. An HB polymer composition according to claim 1 in which the polyethylenically unsaturated compound contains a single group of the formula:

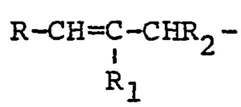

where R and $R_1$ may be the same or different and are selected from hydrogen, lower alkyl or phenyl radicals and

$R_2$ may be hydrogen or lower alkyl, together with at least one different ethylenically unsaturated group.

4. An HB polymer composition according to claim 3 in which the polyethylenically unsaturated compound contains at least one group of formula:

$$R-CH=CR_1-\underset{\underset{O}{\|}}{C}-O-$$

where R and $R_1$ are as previously specified.

5. An HB polymer composition according to claim 1 in which the polyethylenically unsaturated compound contains at least three groups of formula:

$$R-CH=\underset{\underset{R_1}{|}}{C}-\underset{\underset{O}{\|}}{C}-O-$$

wherein R and $R_1$ are as previously specified.

6. An HB polymer composition according to claim 1 in which the polyethylenically unsaturated compound contains at least two maleimido groups linked by a divalent aliphatic, aromatic or cycloaliphatic radical.

7. An HB polymer composition according to either of claims 1 or 2 in which the polyethylenically unsaturated compound is a substituted amide.

8. An HB polymer composition according to any one of claims 1 to 7 in which the polyethylenically unsaturated compound has a melting point which is within the temperature range from about 20°C less than the melting point of the HB polymer to 20°C above the melting point of the HB polymer.

9. An HB polymer composition according to any one of claims 1 to 8 in which a free radical generating agent is present.

10. An HB polymer composition according to any one of the preceding claims in which the composition contains from 0.1 to 10% by weight of the composition of the polyethylenically unsaturated compound.

11. An HB polymer composition according to claim 9 containing from 0.01 to 2.0% by weight of the composition of a free radical generating agent.

12. An HB polymer composition which has been prepared by reacting an HB polymer with at least one polyethylenically unsaturated compound containing at least two reactive groups of the formula $-CH{=}CR_1-$ in which $R_1$ is hydrogen, a lower alkyl or a phenyl radical and in which compound $R_1$ may differ from one $-CH{=}CR_1-$ group to another with the proviso that where the compound contains only two groups of the formula $-CH{=}CR_1-$, they are not both contained in:

$$R{-}CH{=}CR_1{-}\underset{\underset{O}{\|}}{C}{-}O{-}$$

groups where R is hydrogen, a lower alkyl, or a phenyl radical.

13. A polymer composition containing a microbiologically produced HB polymer and having a melt flow time which is at least double that of the HB polymer itself.

0052946

European Patent Office

# EUROPEAN SEARCH REPORT

Application number EP 81 30 5033

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 87, no. 24, 12 December 1977, page 41, abstract 185533p Columbus, Ohio (US)<br>& JP - A - 77 73 948 (FURUKAWA ELECTRIC CO. Ltd)<br>* Whole document *<br>---- | 1 | C 08 L 67/04 |

TECHNICAL FIELDS SEARCHED (Int.Cl. 3)

C 08 L 67/04
C 12 P 7/62
C 08 K 5/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 01-03-1982 | DECOCKER |

EPO Form 1503.1 06.78